# EUROPEAN PATENT SPECIFICATION

(11) **EP 3 974 062 B1**
(45) Date of publication and mention of the grant of the patent: **11.02.2026**
(21) Application number: 20198966.2
(22) Date of filing: 29.09.2020
(51) Int. Cl.: B03C 3/47, B03C 3/08, B03C 3/12, B03C 3/16, B03C 3/41, B01D 53/32

(54) **APPARATUS FOR ELECTROSTATIC DE-ACTIVATION AND REMOVAL OF HAZARDOUS AEROSOLS FROM AIR**
VORRICHTUNG ZUR ELEKTROSTATISCHEN DEAKTIVIERUNG UND ENTFERNUNG VON SCHÄDLICHEN AEROSOLEN AUS LUFT
APPAREIL DE DÉSACTIVATION ÉLECTROSTATIQUE ET D'ÉLIMINATION D'AÉROSOLS DANGEREUX DANS L'AIR

(43) Date of publication of application: 30.03.2022
(73) Proprietor: Davydova, Elena, 6004 Luzern (CH)
(72) Inventor: Fluhrer, Helmut, 8955 Oetwil an der Limmat (CH); Davydova, Elena, 8955 Oetwil an der Limmat (CH); Saveliev, Yuri, Phayao (TH)
(74) Representative: Kurig, Thomas

(56) References cited:
- DE-A1- 3 307 999
- DE-U- 7 520 512
- US-A- 2 523 618
- US-A- 5 843 210
- US-A1- 2003 079 608
- US-A1- 2015 135 949

## Description

### Field of the invention

The present invention relates to an apparatus for electrostatic de-activation and removal of biologically hazardous and other aerosols from air, specifically for air cleaning and / or purification and disinfection by combining air ionization and electro-spraying.

### Prior Art

Aerosol removal from air has become an important aspect in times of bacteria or virus pandemics. There are many applications for the removal of pathogenic bio-aerosols recently discovered to be a primary source and reason of Covid-19 pandemic. There is a concern that the humanity may face pandemics of other airborne transmitted pathogens in the future. Many viruses including SARS-CoV-2 (Covid-19) are small in size (60-125nm). Most of them are released to the air by infected humans and possibly by other sources and are in the form of droplets in a wide spectrum of sizes. Large droplets are sedimented within a short distance from an infected person, but smaller ones evaporate quickly and evaporation residues that contain viruses may stay in the air for hours. In particular, this is especially a problem in enclosed premises with limited ventilation and recirculated air. At the moment, there is no reliable data on the size spectrum of human-produced evaporation residues. However, it is known that only some special air filters (e.g. so-call N95 technology) are able to catch particles with sizes starting from about 300nm and may provide a reasonable degree of protection. Although those filters are widely used in face masks, their application for purifying large volumes of air is highly cumbersome. High costs of sub-micron particle filters are prohibitive in large applications; the filters should be regularly changed and / or replaced which is a dangerous procedure because of a high risk of dissemination of the collected microbes. Their pre-treatment with UV light has been proposed but this brings about an extra degree of complexity and extra costs. Moreover, air filters introduce a high pressure drop at high air flows inevitable in large applications, resulting in a high energy consumption.

It was reported in a number of publications that many microbial species were partly de-activated by negative air ions with different de-activation fractions for different species. Air ions are small molecular clusters with the sizes of about few nanometers and 1-2 electronic charges. Although dielectric barrier discharge (DBD) systems have also been proposed for this purpose (e.g. Xia et al., 2019), the common way to produce air ions of a certain electric sign is by direct current (DC) corona discharge from an emitter electrode, which is in general an electrically conductive object with one or more sharp features (i.e. a part with a low radius of curvature) such as one or more electrically coupled needles or thin wires. The emitter electrode of corona discharge is electrically coupled to the active electrode of a high voltage direct current (HVDC) source, the second electrode of which is grounded. The produced air ions with the sign of the active electrode of HVDC are repulsed and drift from the emitter electrode, attaching to and thus electrically charging aerosol particles in the air, in particular bio-aerosols. The mechanism and probably multiple mechanisms of the de-activation of micro-organisms by air ion attachment are not fully understood yet.

As for SARS-CoV-2 in particular, the de-activation efficiency of this pathogen by air ionization in terms of survival rate at given conditions has not been studied yet. However, it is understood that the air disinfection by ionization is not a reliable solution per se. Alternatively or additionally, the physical removal of pathogens from the air could be a more viable solution. Moreover, it is believed that under some conditions the direct inhalation of ionized air could be dangerous for health. The first problem is that negative corona discharge produces ozone and nitrogen oxides which are poisonous gases. Therefore, a particular corona discharge device should be properly designed to make sure that concentrations of those gases do not exceed the limits imposed by safety regulations. Health benefits of negative ions were reported in a number of publications. However, in some cases, including the case of microbial air contamination under consideration, air ionization of either electric sign may bring another problem, which is the attachment of aerosols including microbes, allergens and other harmful particles to the surfaces of respiratory tract and to the lungs in particular, is enhanced by particle electrification. This is due to the attractive electric force between a charged aerosol particle and its image charge produced on an organ. This effect may be significant even for nano-particles carrying only several electronic charges (e.g. Cohen et al., 1996; Fews et al., 1999). Because the de-activation of pathogenic aerosols by air ions is only partial, the deposition of the remaining active pathogens in the respiratory tract may be higher than in the absence of air ionization. Therefore, for the sake of safety with uncertain total effect of the de-activation and deposition, charged aerosols should be removed from the air before it enters the respiratory tract. Electrostatic precipitators (ESPs) that typically comprise an emitter electrode of corona discharge and electrodes on which charged aerosol particles are collected by attractive electric sources have been proposed for this purpose.

ESPs have been used for the removal of relatively large bio-aerosols such as pollen, fungi and some bacteria (e.g. Alonso et al., 2016). Some viruses have relatively large sizes such as filamentous virions. Influenza A is one of such virions, which has elongated shape with the diameter 80-100nm and variable length of several µm. Hagbom et al. (2015) successfully de-activated (>97%) and collected (up to 21%) influenza A by a small ESP after 40 min. in a small chamber (19 m3), which prevented 100% (4/4) of guinea pigs from infection. However, ESPs are not effective for nano-particles at high process air flows (i.e. the volume of air passed per unit time). Because of the direct relationship between aerosol particle size and the maximum electric charge that it can achieve, de-activation and collection of smaller particles may be problematic. For example, 100 nm particles can achieve about 5 - 10 electronic charges as reported in different literature (e.g. Marquard, 2007). Sufficient charging of nano-particles for this purpose is possible only in the presence of sufficiently strong electric field, the case called field charging. This takes place only at very short distances from the emitter electrode of corona discharge and may take some time of up to about 2 seconds to fully charge nano-particles. Limited volume of charging zone and relatively long charging time make ESPs impractical with high process air flows. In practice, the use of ESPs for small particles is limited to air sampling devices. Safe cleaning of ESP collector electrodes from the aggregated contagious microbes is another technical challenge.

Another approach to the problem of aerosol removal from air is based on their scavenging by a large surface of a body of water or other liquid with a high surface-to-volume ratio, the method called wet scrubbing. Air motions, especially turbulent, and the Brownian motions of aerosol particles significantly promote this process. In wet scrubbing, biologically hazardous aerosols are handled in liquid suspension, which is easier and safer than in the dry form. They can be de-activated in liquid by many means such as heating, using disinfectors, dissolving ozone, exposing to strong corona discharge etc. In many cases, after sedimentation or flocculation of the collected particles, the working liquid can be re-used. Alternatively, the working liquid is replaced and its recycling can be done externally.

There are three main types of wet scrubbers that have been reported in the literature, ie falling film, packed bed or porous pad and spray tower. In falling film towers, the liquid flows in the form of thin films over the solid surfaces, and the process air (i.e. air that is passing through the system, typically with the aid of fans) comes in direct contact with these thin liquid films. The solid surfaces can be tubes or plates, generally arranged in vertical direction. High construction costs and bulky size are the main drawbacks of these towers. In the packed bed towers or porous pads, the process air comes in direct contact with the liquid on large surface of the packing or pad wetted by the liquid flowing downwards. Compared to falling film towers, much higher surface-to-volume values can be achieved in compact systems at much lower capital costs. The main disadvantage of packed bed towers and porous pads is a high pressure drop of process air, especially at high process air flows. In particular, this may lead to high capital and power consumption costs by fans. Another disadvantage of such systems is the maintenance that may be required to clean or replace beddings or pads due to the clogging by scavenged aerosols that are not completely removed by liquid flow and can be accumulated over the time.

In spray towers, air and liquid interact on the surface of small liquid droplets that are typically produced by spray nozzles. In typical spray tower designs, the process air flows upwards, and the liquid droplets from one or more spray nozzles close to the top of the tower, sediment to the bottom. Compared to other abovementioned types of wet scrubbers, practically negligible air pressure drop at very high achievable values of surface-to-volume ratio, low capital and operational costs are main advantages of spray towers. Those advantages make spray towers more suitable in larger applications where higher process air flows should be treated. Smaller droplets have a higher aerosol removal efficiency, but their production requires higher hydraulic liquid pressure and smaller orifices of nozzles, which are more prone to the blockage by solid particles that may be suspended in the liquid. Therefore, the liquid should be well filtered, sedimented or flocculated, especially if re-used as mentioned above. Moreover, smaller droplet sizes should be carefully balanced with lower velocities of process air to allow droplet sedimentation.

The main disadvantage associated with spray towers is that some droplets, especially the smallest ones from the droplet size spectrum, may exit the system due to their carryover by process air. In order to minimize the carryover, severe limitations on droplet size spectrum and the process air flow should be imposed. For any type of spray nozzle, the droplet size spectrum is poly-dispersed. Moreover, smaller water droplets may evaporate, and their evaporation residues can even easier escape with process air. Due to those factors, scaling up of spray towers for high process air flows can be problematic. To combat the problem of droplet carryover, Kumar et al. (2011) proposed mesh packings of spray towers. A significant improvement of performance, which authors claimed as "zero carryover" have been achieved. However, at higher air flows, the inhibition of droplet carryover may come at the cost of the increased pressure drop.

A simple and efficient approach to produce droplets is electro-spraying which is based on liquid atomization by electrical forces. The electro-spraying nozzle is usually made in the form of a capillary where the liquid exiting it is exposed to a strong electric field. For water and other electrically conductive liquids, this is typically achieved with a high voltage charging electrode placed in the vicinity of and acting on the electrically earthed liquid, wherein the electric sign of charged droplets is opposite to the polarity of this electrode. The shear stress on the liquid surface, due to the established electric field, causes the elongation of liquid jet and its disintegration into droplets. The produced droplets can be electrified by this inductive charging to a degree when electrostatic forces overcome the surface tension, causing the fragmentation of droplets into smaller ones. This process, known as Rayleigh instability (Rayleigh,1882), may repeat many times and the droplets produced by electro-spraying can be extremely small, with sizes about 1 µm and even smaller in some cases. Another advantage of the electro-spraying is that droplets are highly charged, up to a fraction of the Rayleigh instability limit.

The charge and size of the droplets can be controlled to some extent by adjusting the liquid flow rate and the voltage applied to the nozzle electrode. Charged droplets are self-dispersing in the space due to mutual Coulomb repulsion, which results in the inhibition of droplet agglomeration by coalescence, which is a common problem with conventional spraying nozzles. In commercial electro-spraying nozzles, depending on a particular design and operating voltage, droplets with the diameter ranging between 10 µm and 100 µm can be easily produced at a low water pressure, nozzle orifice of 0.5 - 1 mm, and the electrode consumption current of several micro amperes per nozzle.

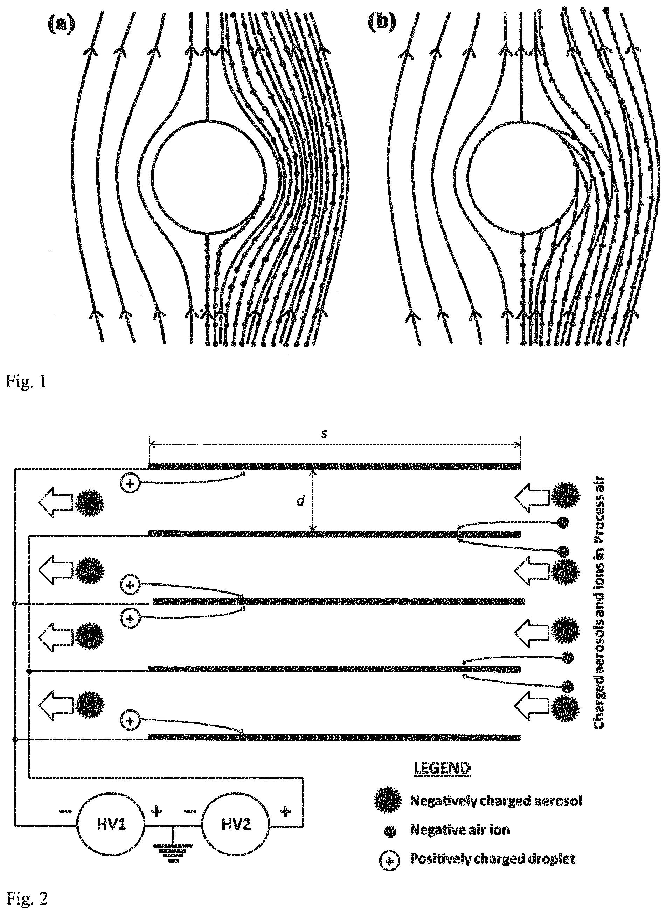

Electric charges on droplets and / or aerosol particles my significantly enhance aerosol scavenging by attractive electric forces. This process called electro-scavenging in some literature, plays an important role in cloud microphysics by promoting ice production in super-cooled clouds (i.e. those at temperatures below 0 °C), which affects weather and climate patterns (e.g. Tinsley et al., 2000; Jaworek et al., 2002). Contact ice nucleation may occur when a solid aerosol particle is scavenged by the descending super-cooled droplet, which may statistically lead to the freezing of the latter. The effect of attractive electric forces on trajectories of aerosol particles near a descending droplet is shown in Fig. 1.

Uncharged aerosol particles flow around the droplet (Fig. 1a). This is especially problematic for smaller particles in conventional spray towers. In the presence of attractive electric forces, particles cross air streamlines (Fig. 1b), which enhances aerosol scavenging.

Tepper at al. (2007) proposed experimental design of spray electro-scrubber where aerosol particles were scavenged by electro-spray droplets due to the charge-to-dipole attractive force between a charged droplet and aerosol particle polarized in the electric field of the neighboring droplet (Jaworek et al., 2002). Then the droplets evaporated leading to the formation of solid residues, which are aggregates of scavenged aerosols retaining a significant electric charge. Those residues can be effectively removed by an ESP, thus solving the problem of droplet carryover at nearly zero pressure drop.

Although the total aerosol removal efficiency of spray electro-scrubber proposed by Tepper at al. (2007) is high, removal efficiencies of aerosol particles with different sizes are different because the droplet-induced dipole moment of a particle and thus the attractive force are proportional to the particle size. Therefore, electro-scavenging of uncharged nano-particles is less effective and thus their removal efficiency is lower compared to that of larger particles. However, introducing even as small as several electronic charges to aerosol particles of the opposite to droplet's sign can significantly enhance the electro-scavenging due to additional long-range Coulomb attractive force (e.g. Jaworek et al., 2002). As previously mentioned, virus-size particles can achieve only about several electronic charges. However, due to high electric charges on electro-spray droplets, relatively high values of the resulting Coulomb force can solve and the problem of nano-particle electro-scavenging. It is preferable that the polarity of DC corona discharge for aerosol charging is negative, so the droplet electric sign is positive. One of the reasons is that negative ions are more efficient in de-activation of some fraction of microbial bio-aerosols at this stage. Another reason for this will be discussed later.

However, the introduction of aerosols charged by air ionization to process air laden with electro-spray droplets of the opposite sign has several technical problems. The first problem is that only a small fraction of air ions produced by corona discharge is attached to aerosols in typical air conditions. Compared to charged aerosols, air ions have a high electrical mobility and their motion may be highly influenced by electric field. Therefore, those of the opposite sign will quickly attach to highly charged droplets resulting in almost instant charge loss of the latter before aerosols can reach droplets. Therefore, air ion concentration in the process air should be minimized as much as possible before the interaction with charged droplets. In principle, it can be achieved with an air ion collector electrode while the motion of charged aerosols with low electrical mobility is mostly directed by process air.

Other problems are related to droplet evaporation. A highly charged droplet will keep most of the original charge during its evaporation only up to a certain point, at which the electric field strength at the droplet surface will exceed the electrical breakdown threshold of the air (in case of typical electro-spray droplet sizes, this is the Paschen limit). This will initiate corona discharge leading to the droplet charge loss and the production of air ions of the droplet sign. This process will continue until the droplet evaporates. In addition to the droplet charge loss, air ions of droplet sign may case a rapid loss of the opposite sign charge on nano-particle aerosols. Another issue is that the vapor density gradient around evaporating droplet is negative and the diffusophoretic force on aerosol particle is positive (away from the droplet), which will further inhibit aerosol scavenging, especially of small particles. Moreover, air ions and gases produced by corona discharge of evaporating charged droplets may be hazardous to the health as mentioned above. In the recommended case of positively charged droplets, positive ions especially problematic because their physiological effects on human and animals are detrimental (even if in sterile air) which was reported in the literature. Another problem with droplet evaporation is that it may cause a noticeable increase in the humidity of indoor air. Although Tepper at al. (2007) argued that this increase is not significant, this may not be true in many cases. Another good reason for the evaporation inhibition and collection of droplets rather than their evaporation residues is handling pathogens as liquid suspension is preferred as mentioned previously.

Patent Literature US 2003/079608 A1 relates to an apparatus for removing particles from air, including an inlet for receiving a flow of air, a first chamber in flow communication with the inlet, wherein a charged spray of semi-conducting fluid droplets having a first polarity is introduced to the air flow so that the particles are electrostatically attracted to and retained by the spray droplets, and an outlet in flow communication with the first chamber, wherein the air flow exits the apparatus substantially free of the particles.

The object of the invention is to provide an improved apparatus for aerosol removal which proves to be efficient for absorbing contaminated aerosols, such as aerosols containing virus or bacteria loads.

### Summary of the invention

The object is solved by an apparatus for electrostatic removal of biologically hazardous and other aerosols from air according to claim 1. Preferred embodiments are mentioned in the subclaims. All other embodiments do not form part of the invention but represent background art that is useful for understanding the invention.

The invention is now illustrated by non-limiting examples in connection with the drawings.

### Brief description of the drawings

Examples are described with reference to the following drawings, wherein
Fig. 1 shows Aerosol particle trajectories near a descending droplet in the absence (a) and presence (b) of attractive electric forces (Tinsley et al., 2007),
Fig. 2 shows an electrostatic collector for ions and charged droplets, and
Fig. 3 shows a schematic diagram of the apparatus for electrostatic removal of biologically hazardous and other aerosols from air in accordance with the invention.

### Detailed description of the drawings

In Fig. 1, the effect of attractive electric forces on trajectories of aerosol particles near a descending droplet is shown. In Fig. 1a, particles flow from bottom to top on air streamlines, wherein only particles get in contact with the droplet, because no "go around" is possible anymore. If there exist attractive electric forces as shown in Fig. 2, that means the droplet and particles are electrically charged, particles can cross air streamlines and get in contact with the droplet.

In Fig. 2, the collector zone of the present invention, an electrostatic collector for ions and charged droplets can be seen. Two or more electrodes (five of them are shown in Fig. 2 for illustration purposes) are arranged in sequence parallel to each other with a distance d and a length s, wherein even electrodes are electrically coupled to the first high voltage direct current source and odd electrodes are electrically coupled to the second high voltage direct current source of the opposite polarity and operating at the same voltage as the first one. Grounding is shown between the two high voltage direct current sources. When negatively charged aerosols and negative ions in the air flow pass the electrodes, the negative air ions will be collected by the positively charged electrodes. Further, also a small fraction of negatively charged aerosols can be collected at the same time. Most of the negatively charged aerosols continue to drift with the air flow.

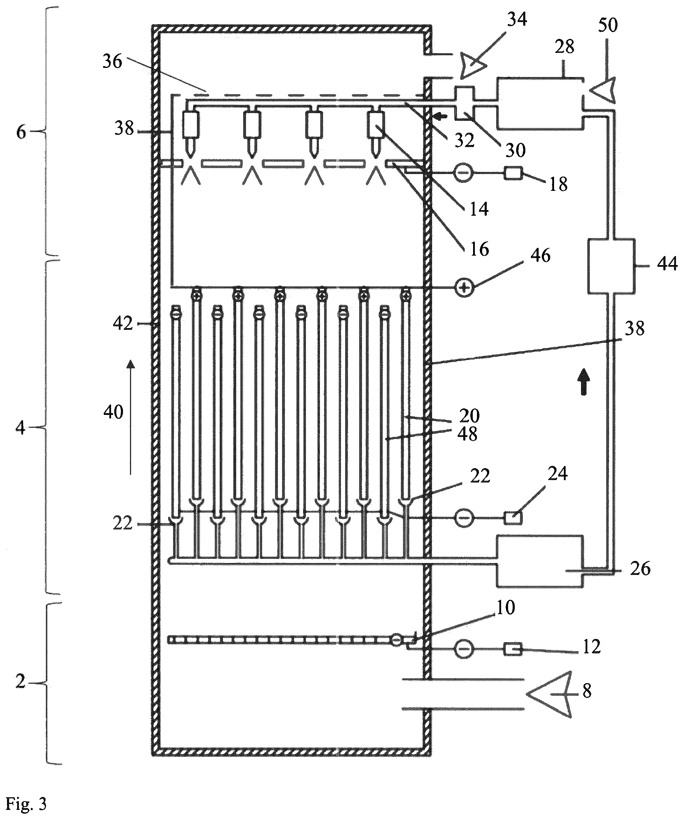

Further, it is shown in Fig. 2, that positively charged droplets are collected by the negatively charged electrodes. As it will be illustrated in Fig. 3, the positively charged droplets are pushed against the air flow direction and on their way the negatively charged aerosols, which continued to drift with the air flow, are absorbed by the droplets.

The collector comprises a series of conductive plates parallel to each other which are electrically coupled in alternate order in sequence to active electrodes of positive and negative high voltage direct current sources of the same operating voltage. The number of positive and negative plates is the same and the collector acts as a stack of plate capacitors with zero total electric charge where high strength electric field is maintained between each couple of neighboring plates.

In such arrangement, different particles with different electric sign can be collected by attractive electric force directed toward either sign plate depending on the particle sign. The collection distance s should be at least the longest of collection distances of all kinds of particles under consideration, i.e. charged droplets and air ions. In this particular case, most of aerosol particles will pass through the collector with process air and charged droplets will take more time to reach a plate electrode than air ion; therefore, the distance s is the largest of the droplet spectrum. It is preferred that the plates arranged vertically or at a small angle to the normal to the ground to facilitate the run-off of working liquid from wetted electrodes by dripping.

In Fig. 3, a configuration of the proposed apparatus is illustrated, which is preferably positioned vertically. In the lower portion of the apparatus, an ionization zone 2 is arranged, above a collector zone 4 (as described partly already for Fig. 2) and above the collector zone 4 a spraying zone 6 is arranged. The parts of all three zones are located in a non-conductive housing 42 with closed bottom and closed top and made from non-electrically conductive material, preferably having a square or rectangular cross section.

The air, which contains hazardous biological or other aerosols, in other words contaminated air with particles of different sizes, enters the apparatus for air cleaning and / or purification and disinfection through an air inlet 8 in ionization zone 2. The optimal liquid flow is about 0,2-1,0 cm³/sec per nozzle, preferably 0,35-0,45 cm³/sec with a process air speed of 1,5-2,5 m/sec, preferably 1,75-2,25 m/sec. The air stream is guided to an emitter electrode of negative corona discharge 10, which is made of parallel segments of thin wire or a wire mesh in planar configuration parallel to the ground with sharp features which does not create a significant pressure drop. A high-voltage direct current source 12 having negative polarity in the preferred case illustrated in Fig. 3 is electrically coupled to emitter electrode 10 for providing energy for ionizing the air stream by the effect of the electrode 10.

The ionized air stream continues to flow to collector zone 4. In the collector zone 4, a sequence of parallel metal plates called electrodes are arranged vertically with the same preferred length of 0,5-2 m and spaced apart from each other with a distance depending on the used voltage, wherein in the non-limiting example of Fig. 3, 12 plates are provided. 6 electrically coupled plates 20 are charged by a high-voltage direct current source having positive polarity 46 and the other 6 electrically coupled plates 48 are charged by a high-voltage direct current source having negative polarity 24, which means it constitutes an electrically neutral system. All positively charged plates 20 are vertically offset by a certain distance with respect to the negatively charged plates 48. Below each plate 20, 48 a collection gutter for dripping liquid is installed. All collected liquid is transported through a pipe or hose to a collection tank 26, which is located outside the housing 42. During the flow of air stream through plates 20, 48 in air flow direction 40, negatively charged aerosols and negative ions in the air flow pass the electrodes, wherein all the negative air ions will be collected by the positively charged electrodes 20. Further, also a small fraction of negatively charged aerosols can be collected at the same time, but most of the negatively charged aerosols continue to drift in air flow direction 40.

In spraying zone 6, 4 electro-spraying nozzles 14 in this non-limiting example, which are arranged side by side, produce a plurality of downward directed jets of positively charged droplets. The electro-spraying nozzles 14 create droplets with a diameter of 20-100 µm, which are pushed through a charging electrode 16 to acquire high positive electric charges, which power is supplied by a high-voltage direct current source 18 of the negative polarity. Between the charging electrode 16 and the plates 20, 48, there is a cavity which vertically extends 0,25-2 m where the droplets can effectively absorb weakly charged aerosol nano-particles such as viruses, which could not be collected by the positively charged electrodes 20 before. These remaining charged aerosols mainly have small sizes and are weakly charged. After the positively charged droplets have absorbed the remaining negatively charged aerosols, they are flowing together through collector zone 4 with the help of gravitational forces plus spraying forces, where the positively charged droplets are collected by the negatively charged electrodes 48. Then, the droplets collected by the plates 20, 48 are to be collected with narrow gutters 22 below and along the plates 20, 48 at a small angle relative to the ground and are transported through a pipe or hose to a collection tank 26.

Further, the material of the electro-spraying nozzles 14 can be either electrically conductive or non-conductive but highly corrosion resistant in case of salt solutions as hygroscopic working liquid. This liquid is supplied to nozzles from a storage tank 28 by means of a hydraulic pump 30 through a pipe or hose 32. The storage tank 28 and the hydraulic pump are located outside the housing 42.

The working liquid is preferably the aqueous solution of a salt at relatively high concentrations affecting the equilibrium relative humidity (ERH), in which microbes are de-activated after a certain time. Additionally, salt solution deactivates pathogens and suppresses evaporation of water. The working liquid is supplied to the storage tank 28 via an external supply or the liquid from the collection tank 26 is taken for recycling off-site with a regeneration unit 44 and, after this process, returned to the storage tank 28.

At the top of the apparatus, the air flow is leaving the apparatus through a metal mesh 36, which is electrically coupled by a connecting wire 38 to high-voltage direct current source 46 via electrode (20) in the case shown in Fig. 3 or to an additional high-voltage direct current source (not shown).

The reason for movement of charged aerosols and ions in the described manner above is the high positive space charge of the electro-spray droplet laden air above collector zone 4. Thereby, air ions will drift upward together with charged aerosols in the process air until they reach electrostatic collector and terminate on its positive plates 20, while most of negatively charged aerosols will pass the collector and enter droplet-laden absorbing zone. On the other hand, positively charged droplets will experience downward electric force in the electric field of negative space charge in the ionization zone 2. This force which is additional to gravitational one and even could be dominant allows smaller droplet size and / or higher air process flow in the system. Eventually, droplets with absorbed aerosols will reach electrostatic collector and deposit on its negative plates.

The process proposed by Tepper at al. (2007) can be significantly improved according to physical principles and recommendations of the present invention.

In this invention, a multiple stage process along the process air, which combines a number of synergetic physical mechanisms, is proposed. In the first stage, aerosols in process air flow are negatively charged with an emitter electrode of corona discharge. In the second stage, air ions and possibly a small fraction of charged aerosols are removed from the air by specially designed multi-electrode electrostatic collector. In this stage, practically all negative ions can be removed from process air, while most of charged aerosol particles continue to drift with this air (it will be discussed in more details later). In the third stage, working liquid is atomized into small positively charged droplets (20 - 100 µm diameter is preferred) by one or more electro-spray nozzles (operating at DC in the range of 5 - 10 kV depending on configuration) and introduced in this form into process air, where the droplets can effectively scavenge weakly charged aerosol nano-particles such as viruses. It is preferred that working liquid is hygroscopic to prevent the droplet evaporation and maintain the relative humidity (RH) in a premise in the optimal range. In the preferred configuration, the direction of droplet ejection by nozzles is opposite to the direction of process air. In the fourth stage, droplets of the suspension of scavenged aerosols in working liquid are electrostatically collected. It is preferred that the electrostatic collector for liquid droplets is the same as the collector used for ion collection in the second stage and comprises negative and positive electrodes electrically coupled to negative and positive active electrodes of two separate HVDC sources, which other electrode is earthed. The suspension accumulated on wetted collector electrodes drips into trays to avoid continuous shortcutting stream and then directed from trays to the first storage tank where the collected bio-aerosols are de-activated and from which the suspension is further directed to one or more sedimentation tanks, where the collected aerosols are sedimented. After the sedimentation, the purified working liquid can be re-used.

Evaporation of working liquid can be inhibited by using liquid desiccants that are typically aqueous hygroscopic salt solutions instead of water. Droplets of such solutions will not evaporate at the relative humidity (RH) of air equal to the equilibrium relative humidity (ERH) of salt solution. In the case of RH > ERH, the water vapor in air will condense on droplets and the solution droplets will evaporate in the opposite case. According to recent studies, maintaining RH in the optimal range is more important than previously thought. The airborne transmission of the SARS-CoV-2 via aerosol particles in indoor environment seems to be correlated with RH. An indoor relative humidity in the range of 40% to 60% cent could reduce the spread of this virus (Ahlawat et al., 2020). Although the additional benefit of this factor could be minor due to the removal of this pathogen particles, the safest RH range stated by authors is about the same as recommended for general health. The value of ERH of a particular salt solution or any other desiccant mostly depends on the chemical composition and concentration of a particular desiccant or a mixture of desiccants. In addition to air purification, electro-spraying of fine salt solution droplets can bring the benefit of RH stabilizing in premises within the optimal range.

Another benefit of salt solutions is that they are generally not favorable media for microbial pathogens. To achieve the recommended ERH of about 50%, the salt concentration should be high enough to inhibit or even permanently de-activate them. The information on this subject for different pathogens and different salts is still scarce at this stage of the art. Quan et al. (2017) reported the effectiveness of table salt (NaCl) in air filters in the inactivation of viruses. Seo et al. (2012) investigated the resistance of murine norovirus (MNV) and coliphage MS2, a culturable human norovirus surrogate, to temperature, salt, and pH. Both MNV and MS2 were rapidly inactivated at temperatures above 60°C. MNV demonstrated a high sensitivity to salt concentrations as low as 3.3 to 6.3% NaCl.

Although there is uncertainty about the de-activation time of different pathogens in different salt solutions at different concentrations, it is conservatively assumed to be within an hour, probably in conjunction with a moderate heating, before the solution can be re-used. Casual maintenance may be required to remove accumulated mud from sedimentation tank and keep the salt concentration within an optimal limit.

Even if the risk of leaking of uncollected droplets into the respiratory tract is very small, safety regulations in many countries require that the selected salt is not toxic. The ERH of non-toxic saturated magnesium chloride solution is about 33% which makes it a good candidate for this application. In practice, the waste sea brine from desalination plant, which is reach in Mg and Ca chlorides after removal or reducing of NaCl by industry standard processing is a cheaper alternative with the ERH of saturated solution of about 34%. The treated sea brine is natural and the degree of NaCl removal and concentration can be adjusted to the required ERH.

It is not necessary to limit ozone and nitrogen oxides production by corona discharge in ionization zone by safety standards, which limits corona discharge intensity in terms of ion production rate. This is because those gases in process air will quickly dissolve in droplets with high surface-to mass ratios. Ozone is a well-known efficient and rapid de-activator of micro-organisms, which will act together with salt solution as working liquid.

### List of reference signs

- 2: ionization zone
- 4: collector zone
- 6: spraying zone
- 8: air inlet
- 10: emitter electrode of corona discharge
- 12: high-voltage direct current source having negative polarity
- 14: electro-spraying nozzle
- 16: charging electrode
- 18: high-voltage direct current source having negative polarity
- 20: electrode
- 22: gutter
- 24: another high-voltage direct current source having negative polarity
- 26: liquid collection tank
- 28: storage tank
- 30: hydraulic pump
- 32: pipe or hose
- 34: air outlet
- 36: metal mesh
- 38: connecting wire
- 40: air flow direction
- 42: housing
- 44: liquid regeneration unit
- 46: another high-voltage direct current source having positive polarity
- 48: other electrode
- 50: external supply

## Claims

1. Apparatus for electrostatic removal of biologically hazardous and other aerosols from air, said apparatus located in a housing (42) made of non-electrically conductive material, comprising
an ionization zone (2) located in the housing (42) and configured for charging aerosols contained in a stream of supplied air,
a spraying zone (6) located in the housing (42) and configured for absorbing said charged aerosols with positively charged droplets, and
a collector zone (4) located in the housing (42) and configured for removing negative air ions and said charged aerosols from said stream of supplied air and said positively charged droplets, wherein said collector zone (4) is located between said ionization zone (2) and said spraying zone (6) and comprises an electrostatic collector,
wherein said ionization zone (2) comprises at least one air inlet (8) for guiding said stream of supplied air to an emitter electrode of negative corona discharge (10) located in said ionization zone (2), wherein the apparatus comprises a first high-voltage direct current source (12) and said emitter electrode (10) is connected to negative polarity of said first high-voltage direct current source (12), wherein said spraying zone (6) comprises at least one electro-spraying nozzle (14) for ejecting droplets opposite to an air flow direction (40) in said collector zone (4) and at least one charging electrode (16) arranged adjacent to said at least one electro-spraying nozzle (14) for electrically charging said droplets, wherein the apparatus comprises a second high-voltage direct current source (18) and said charging electrode (16) is connected to negative polarity of said second high-voltage direct current source (18), in which said connected polarity that said charging electrode (16) is connected to have the same polarity as said high-voltage direct current source (12) to which said emitter electrode (10) is connected,
wherein said electrostatic collector further comprises a plurality of electrodes (20, 48), in the form of positively charged parallel plates (20) and negatively charged parallel plates (48), having charges of alternating positive and negative electric signs with the same absolute value and which are oriented towards said air flow direction (40), a gutter (22) located under each electrode for collecting dripping liquid, the gutter (22) being hydraulically connectable to at least one liquid collection tank (26), wherein said negatively charged parallel plates (48) are charged by a high-voltage direct current source having a negative polarity (24) and said positively charged parallel plates (20) are charged by a high-voltage direct current source having a positive polarity (46), so as to constitute an electrically neutral system.

2. Apparatus according to claim 1, wherein said air inlet (8) comprises at least one fan.

3. Apparatus according to any of claims 1 or 2, wherein said emitter electrode of corona discharge (10) is formed of parallel segments of wire or a wire mesh in planar configuration.

4. Apparatus according to any of claims 1 to 3, wherein said emitter electrode of corona discharge (10) is configured to operate at up to 40 kV, preferred between 10-15 kV.

5. Apparatus according to claim 1, wherein dripped drops, which are collected by said gutters (22), are adapted to be collected in said at least one liquid collection tank (26).

6. Apparatus according to any one of claims 1 to 5, wherein said at least one electro-spraying nozzle (14) is configured to produce hygroscopic liquid droplets having an average diameter between 20-100 µm.

7. Apparatus according to any of claims 1 to 6, wherein said at least one charging electrode (16) is configured to operate at 5-10 kV.

8. Apparatus according to claim 1, wherein said spraying zone (6) further comprises an air outlet (34), wherein said air outlet (34) has an optional metal mesh (36) for outflowing air to pass, and said optional metal mesh (36) is electrically coupled to said high-voltage direct current source having positive polarity (46) via at least one positively charged parallel plates (20) or connectable to an additional high-voltage direct current source having positive polarity having a connecting wire (38).

## Patentansprüche

1. Vorrichtung zur elektrostatischen Entfernung von biologisch gefährlichen und anderen Aerosolen aus der Luft, wobei sich die Vorrichtung in einem Gehäuse (42), das aus elektrisch nicht leitendem Material hergestellt ist, befindet, umfassend
eine Ionisationszone (2), die sich in dem Gehäuse (42) befindet und so konfiguriert ist, dass sie in einem zugeführten Luftstrom enthaltene Aerosole auflädt,
eine Sprühzone (6), die sich in dem Gehäuse (42) befindet und zum Absorbieren der geladenen Aerosole mit positiv geladenen Tröpfchen konfiguriert ist, und
eine Sammelzone (4), die sich in dem Gehäuse (42) befindet und zum Entfernen negativer Luftionen und der geladenen Aerosole aus dem zugeführten Luftstrom und den positiv geladenen Tröpfchen konfiguriert ist, wobei sich die Sammelzone (4) zwischen der Ionisationszone (2) und der Sprühzone (6) befindet und einen elektrostatischen Kollektor umfasst,
wobei die Ionisationszone (2) mindestens einen Lufteinlass (8) zum Leiten des zugeführten Luftstroms zu einer Emitterelektrode einer negativen Koronaentladung (10) umfasst, die sich in der Ionisationszone (2) befindet, wobei die Vorrichtung eine erste Hochspannungs-Gleichstromquelle (12) umfasst und die Emitterelektrode (10) mit der negativen Polarität der ersten Hochspannungs-Gleichstromquelle (12) verbunden ist,
wobei die Sprühzone (6) mindestens eine Elektrospray-Düse (14) zum Ausstoßen von Tröpfchen entgegen einer in der Sammelzone (4) vorherrschenden Luftströmungsrichtung (40) und mindestens eine Ladeelektrode (16) umfasst, die benachbart zu der mindestens einen Elektrospray-Düse (14) angeordnet ist, um die Tröpfchen elektrisch zu laden, wobei die Vorrichtung eine zweite Hochspannungs-Gleichstromquelle (18) umfasst und die Ladeelektrode (16) mit der negativen Polarität der zweiten Hochspannungs-Gleichstromquelle (18) verbunden ist, wobei die Ladeelektrode (16) mit derselben Polarität verbunden ist wie der Pol der Hochspannungs-Gleichstromquelle (12), mit dem die Emitterelektrode (10) verbunden ist,
wobei der elektrostatische Kollektor ferner eine Vielzahl von Elektroden (20, 48) in der Form von positiv geladenen parallelen Platten (20) und negativ geladenen parallelen Platten (48) umfasst, die Ladungen mit wechselnd positiven und negativen elektrischen Vorzeichen mit dem gleichen Absolutwert aufweisen und die in Richtung des Luftstroms (40) ausgerichtet sind, sowie eine unter jeder Elektrode angeordnete Rinne (22) zum Sammeln tropfender Flüssigkeit, wobei die Rinne (22) hydraulisch mit mindestens einem Flüssigkeitssammelbehälter (26) verbunden ist, wobei die negativ geladenen parallelen Platten (48) durch eine Hochspannungs-Gleichstromquelle mit einer negativen Polarität (24) und die positiv geladenen parallelen Platten (20) durch eine Hochspannungs-Gleichstromquelle mit positiver Polarität (46) geladen werden, um ein elektrisch neutrales System zu bilden.

2. Vorrichtung nach Anspruch 1, wobei der Lufteinlass (8) mindestens einen Ventilator umfasst.

3. Vorrichtung nach einem der Ansprüche 1 oder 2, wobei die Emitterelektrode der Koronaentladung (10) aus parallelen Drahtsegmenten oder einem Drahtgeflecht in planarer Konfiguration gebildet ist.

4. Vorrichtung nach einem der Ansprüche 1 bis 3, wobei die Emitterelektrode der Koronaentladung (10) so konfiguriert ist, dass sie bei bis zu 40 kV, bevorzugt zwischen 10-15 kV, arbeitet.

5. Vorrichtung nach Anspruch 1, wobei die in den Rinnen (22) gesammelte abgetropfte Flüssigkeit in dem mindestens einen Flüssigkeitssammelbehälter (26) gesammelt wird.

6. Vorrichtung nach einem der Ansprüche 1 bis 5, wobei die mindestens eine Elektrospray-Düse (14) so konfiguriert ist, dass sie hygroskopische Flüssigkeitströpfchen mit einem durchschnittlichen Durchmesser zwischen 20-100 µm erzeugt.

7. Vorrichtung nach einem der Ansprüche 1 bis 6, wobei die mindestens eine Ladeelektrode (16) so konfiguriert ist, dass sie bei 5-10 kV arbeitet.

8. Vorrichtung nach Anspruch 1, wobei die Sprühzone (6) ferner einen Luftauslass (34) umfasst, wobei der Luftauslass (34) ein optionales Metallgeflecht (36) zum Durchtritt ausströmender Luft aufweist und das optionale Metallgeflecht (36) entweder über mindestens eine positiv geladene parallele Platte (20) elektrisch mit der Hochspannungs-Gleichstromquelle mit positiver Polarität (46) verbunden ist oder mit einer zusätzlichen Hochspannungs-Gleichstromquelle mit positiver Polarität über einen Verbindungsdraht (38) verbunden ist.

## Revendications

1. Appareil pour l'élimination électrostatique d'aérosols biologiquement dangereux, et autres, présents dans l'air, ledit appareil étant situé dans un boîtier (42) fait en un matériau non conducteur de l'électricité, comprenant
une zone d'ionisation (2), située dans le boîtier (42) et configurée pour charger des aérosols contenus dans un flux d'air alimenté,
une zone de pulvérisation (6), située dans le boîtier (42) et configurée pour absorber lesdits aérosols chargés avec des gouttelettes chargées positivement, et
une zone de collecte (4), située dans le boîtier (42) et configurée pour éliminer, dans ledit flux d'air alimenté, les ions négatifs présents dans l'air et lesdits aérosols chargés, et lesdites gouttelettes chargées positivement, ladite zone de collecte (4) étant située entre ladite zone d'ionisation (2) et ladite zone de pulvérisation (6) et comprenant un collecteur électrostatique,
ladite zone d'ionisation (2) comprenant au moins une entrée d'air (8) pour guider ledit flux d'air alimenté vers une électrode émettrice de décharge corona négative (10) située dans ladite zone d'ionisation (2), l'appareil comprenant une première source de courant continu à haute tension (12) et ladite électrode émettrice (10) étant connectée à la polarité négative de ladite première source de courant continu à haute tension (12), ladite zone de pulvérisation (6) comprenant au moins une buse d'électro-pulvérisation (14) pour éjecter des gouttelettes dans le sens opposé à la direction du flux d'air (40) dans ladite zone de collecte (4) et au moins une électrode de charge (16) agencée à proximité de ladite au moins une buse d'électro-pulvérisation (14) pour charger électriquement lesdites gouttelettes, l'appareil comprenant une deuxième source de courant continu haute tension (18) et ladite électrode de charge (16) étant connectée à la polarité négative de ladite deuxième source de courant continu haute tension (18), dans lequel ladite polarité connectée, à laquelle ladite électrode de charge (16) est connectée, a la même polarité que ladite source de courant continu haute tension (12) à laquelle ladite électrode d'émission (10) est connectée,
ledit collecteur électrostatique comprenant en outre une pluralité d'électrodes (20, 48), sous la forme de plaques parallèles (20) chargées positivement et de plaques parallèles (48) chargées négativement, ayant des charges de signes électriques positifs et négatifs alternés de même valeur absolue et qui sont orientées vers ladite direction d'écoulement d'air (40), une gouttière (22) située sous chaque électrode pour collecter le liquide qui s'égoutte, la gouttière (22) étant apte à être reliée hydrauliquement à au moins un réservoir (26) de collecte de liquide, lesdites plaques parallèles (48) chargées négativement étant chargées par une source de courant continu à haute tension ayant une polarité négative (24) et lesdites plaques parallèles (20) chargées positivement étant chargées par une source de courant continu à haute tension ayant une polarité positive (46), de manière à constituer un système électriquement neutre.

2. Appareil selon la revendication 1, dans lequel ladite entrée d'air (8) comprend au moins un ventilateur.

3. Appareil selon l'une quelconque des revendications 1 ou 2, dans lequel ladite électrode émettrice de décharge corona (10) est formée de segments parallèles de fil ou d'un treillis métallique dans une configuration plane.

4. Appareil selon l'une quelconque des revendications 1 à 3, dans lequel ladite électrode émettrice de décharge corona (10) est configurée pour fonctionner jusqu'à 40 kV, de préférence entre 10 et 15 kV.

5. Appareil selon la revendication 1, dans lequel les gouttes qui s'égouttent, qui sont recueillies par des gouttières (22), sont adaptées pour être recueillies dans ledit au moins un réservoir (26) de collecte de liquide.

6. Appareil selon l'une quelconque des revendications 1 à 5, dans lequel ladite au moins une buse d'électro-pulvérisation (14) est configurée pour produire des gouttelettes de liquide hygroscopiques ayant un diamètre moyen compris entre 20 et 100 µm.

7. Appareil selon l'une quelconque des revendications 1 à 6, dans lequel ladite au moins une électrode de charge (16) est configurée pour fonctionner entre 5 et 10 kV.

8. Appareil selon la revendication 1, dans lequel ladite zone de pulvérisation (6) comprend en outre une sortie d'air (34), ladite sortie d'air (34) présentant un treillis métallique optionnel (36) pour laisser passer l'air sortant, et ledit treillis métallique optionnel (36) étant relié électriquement à ladite source de courant continu haute tension de polarité positive (46) via au moins une plaque parallèle chargée positivement (20), ou étant apte à être connecté à une source de courant continu haute tension supplémentaire de polarité positive présentant un fil de connexion (38).
